Europäisches Patentamt

European Patent Office

Office européen des brevets

(19)

(11) Publication number: **0 035 815**
**B1**

(12) **EUROPEAN PATENT SPECIFICATION**

(45) Date of publication of patent specification: **08.06.83**

(51) Int. Cl.³: **C 07 C 107/06**

(21) Application number: **81200246.7**

(22) Date of filing: **04.03.81**

(54) Process for the preparation of p-aminoazobenzene from aniline.

(30) Priority: **12.03.80 NL 8001453**

(43) Date of publication of application:
**16.09.81 Bulletin 81/37**

(45) Publication of the grant of the patent:
**08.06.83 Bulletin 83/23**

(84) Designated Contracting States:
**DE FR GB IT NL**

(56) References cited:
**GB - A - 859 221**
**NL - A - 7 703 353**
**US - A - 2 521 095**
**US - A - 2 538 431**
**US - A - 3 349 073**
**US - A - 3 382 228**
**US - A - 4 083 846**

(73) Proprietor: **Akzo N.V.**
**Velperweg 76**
**NL-6824 BM Arnhem (NL)**

(72) Inventor: **de Graaf, Johannes**
**Henri Dunantlaan 31**
**NL-7548 AB Boekelo (NL)**

(74) Representative: **Sieders, René et al,**
**P.O. Box 314**
**NL-6800 AH Arnhem (NL)**

Courier Press, Leamington Spa, England.

# O 035 815

Process for the preparation of p-aminoazobenzene from aniline

The invention relates to a process for the preparation of p-aminoazobenzene which comprises the steps of reacting an excess of aniline with an alkali metal nitrite in a hydrochloric acid medium at a temperature below 100°C, isomerizing the resulting diazoamino compound in the same medium, neutralizing the reaction mixture and separating the resulting aqueous phase from the organic phase which contains the formed p-aminoazobenzene dissolved in aniline.

In US 2 538 431 it is proposed to carry out this preparation in a monohydric alcohol medium while observing strict limiting conditions as to reaction time and temperature.

In GB 859221 the isomerization is performed at a higher temperature level than the diazotization while, depending on the use or not of a Friedel-Crafts catalyst, the acid to nitrite molar ratio is kept within certain ranges.

On a technical scale these known methods do not provide sufficient control over the formation of by-products, of which particularly the so-called phenyl anilines and diazo tar are very bothersome. To suppress this formation of by-products it has been previously proposed in the published patent application NL 7 703 353 to carry out the preparation in the presence of elementary oxygen dissolved in the reaction liquid. Although this provision has some favourable effect, it is considered less attractive in actual practice in view of explosion hazards.

It has now been found that the formation of phenyl anilines and diazo tar may at least equally well or more effectively be suppressed by the presence of certain other additives without the risk of the formation of explosive gas mixtures.

According to the invention the present process is characterized in causing the isomerisation to proceed in the presence of a compound selected from the group consisting of acrylonitrile, methacrylonitrile, methacrylic acid, alkyl methacrylate with 1 to 4 carbon atoms in the alkyl radical, and butadiene in an amount of at least 0.1% by weight, based on the aniline.

The preparation of the p-aminobenzene is effected essentially in two steps.

In the first step diazotization of aniline with nitrite takes place in a hydrochloric acid medium with formation of diphenyl triazine as diazoamino compound. By an acid catalysed reaction this compound is subsequently rearranged or isomerized to p-amino azobenzene and a little of the corresponding ortho compound.

The formation of phenyl anilines and diazo tar is believed to predominantly take place during this rearrangement and the presence of an inhibiting additive is therefore certainly required in that stage of the process. There are no objections at all, however, to having the inhibiting additive present during diazotization.

The diazotization is primarily a reaction between aniline and nitrous acid and the latter reagent is normally generated by mixing an alkali metal nitrite with hydrochloric acid. Alternative means of generating nitrous acid are of course available to the chemist, but seldom of commercial interest.

It has been found that for the present additives to be appreciably effective they should be used in an amount of at least 0,1% by weight, based on the total amount of aniline.

The use of amounts higher than 20% by weight has hardly any further effect and is unnecessarily detrimental to the economy of the process. Preferably, the inhibiting additive is employed in an amount of about 0,5 to 10% by weight, based on the aniline.

Upon neutralization of the acid the p-aminobenzene formed in the present process is obtained as a solute in aniline, which is separated from the aqueous phase.

Depending on its further utilization the p-aminobenzene, containing a few % by weight of o-aminoazobenzene as the only important impurity can be recovered from the aniline in a known manner or be further processed as such in solution. The quality of the aniline solution is so good that the direct processing by catalytic hydrogenation to p-phenylene diamine, an important starting material for the preparation of aromatic polyamides, proceeds satisfactorily.

The process according to the invention is illustrated but not limited by the following examples.

Example I

In 100-ml sample bottles test reactions were carried out with different amounts of acrylonitrile. To this end there were introduced into each bottle, provided with a magnetic stirrer, 5 ml of aniline and 1,6 ml of hydrochloric acid (30%). In addition to a blank experiment different amounts of acrylonitrile were accurately measured into the sample bottles. Subsequently, after adding 1,2 ml of $NaNO_2$ solution in water (40%), the bottles were placed in ice and agitated every now and then. After a few minutes crystals (diphenyl triazene) appeared and the bottles were kept in a thermostatted bath of 35°C for at least 4 hours, with continuous magnetic stirring. The contents of the bottles were neutralized with 0,7—0,8 ml of 33%-sodium hydroxyde solution, after which the aniline layer was analysed by liquid- and thin-layer chromatography. Table A gives the percentages by weight of p-aminoazobenzene (pAAB), o-aminoazobenzene (oAAB), phenyl anilines (FA) and tar found in the aniline.

2

## 0 035 815

### TABLE A

| | ml acrylonitrile added | | | | | |
|---|---|---|---|---|---|---|
| | none | 0,01 | 0,03 | 0,1 | 0,25 | 0,75 |
| pAAB | 32,0 | 30,6 | 30,3 | 30,9 | 31,3 | 26,5 |
| oAAB | 2,30 | 2,19 | 2,48 | 2,44 | 2,28 | 2,52 |
| FA | 0,48 | 0,19 | 0,06 | 0,02 | n.m. | n.m. |
| tar | 0,55 | 0,15 | 0,06 | 0,03 | 0,01 | n.m. |

n.m.=not measurable

Example II

A larger scale experiment was carried out in a 6-l reactor provided with a cooling jacket, a stirrer and a metering device. Into the reactor there were charged 4100 g of aniline, 970 g of 30%-hydrochloric acid and 20 g of acrylonitrile besides adding no acrylonitrile for making a blank determination. Use being made of a connected thermostat, the mixture was heated to 35°C, after which a solution of 830 g of $NaNO_2$ in water (40%) was added with proper stirring, over a period of 1 hour. To complete the isomerization the reaction mixture was kept at said temperature for at least 4 hours. Next, the reaction mixture was neutralized to a pH above 8, after which the brine layer was separated. Upon analysis of the aniline layer the results summarized in Table B were found.

### TABLE B

| | additive | |
|---|---|---|
| | none | acrylonitrile |
| pAAB | 20,9 | 20,7 |
| oAAB | 1,48 | 1,54 |
| FA | 0,08 | 0,01 |
| tar | 0,049 | 0,01 |

Example III

Into a 500-ml flask provided with a stirrer and a dropping funnel there were charged 100 ml of aniline and 30 ml of hydrochloric acid (30%). The temperature of the flask was set to 40°C, after which 10 ml of methyl methacrylate were added, besides adding none for a blank determination.

Next, a solution of 20 ml of $NaNO_2$ in water (40%) was added dropwise over a period of 10 minutes. The reaction mixture was kept at the above temperature for at least 4 hours and subsequently neutralized with 12 ml of sodium hydroxyde solution (33%).

The results of the analysis of the organic reaction product are summarized in Table C.

### TABLE C

| | additive | |
|---|---|---|
| | none | methylmethacrylate |
| pAAB | 23,4 | 23,1 |
| oAAB | 1,97 | 1,91 |
| FA | 0,14 | 0,005 |
| tar | 0,11 | 0,005 |

Example IV

The experiment of Example III was repeated with 0,5 wt.%, based on aniline, of methacrylonitrile as the additive instead of methyl methacrylate. During the 4 hours isomerization period the temperature of the flask was held at 30°C.

Analysis of the organic product layer gave the following weight percentage figures:

3

| | |
|---|---|
| pAAB | 23,9 |
| oAAB | 1,70 |
| FA | <0,01 |
| tar | 0,02 |

Example V

As in Example I three sample bottles were charged with aniline and hydrochloric acid and two of these bottles were further charged with 5 $\mu$l and 50 $\mu$l, respectively, of methacrylic acid. The diazotization was made with 1,0 ml of a 40% $NaNO_2$ solution in water, the remaining procedure being as before.

The analytical data obtained are summarized in Table D.

TABLE D

| | $\mu$l methacrylic acid added | | |
|---|---|---|---|
| | none | 5 | 50 |
| pAAB | 25,1 | 24,6 | 26,3 |
| oAAB | 1,78 | 1,73 | 1,82 |
| FA | 0,15 | 0,09 | <0,01 |
| tar | 0,22 | 0,14 | 0,07 |

Example VI

Example I was repeated with n-butyl methacrylate as the additive. Different amounts of this additive were accurately measured into the sample bottles and the diazotization was made with 1,2 ml of a 38,5% $NaNO_2$ solution in water.

Analysis of the aniline layer gave the percentage data summarized in Table E.

TABLE E

| | $\mu$l n-butylmethacrylate added | | | | | |
|---|---|---|---|---|---|---|
| | none | 5 | 15 | 50 | 150 | 500 |
| pAAB | 28,5 | 28,5 | 29,9 | 29,8 | 30,4 | 28,7 |
| oAAB | 2,36 | 2,07 | 2,14 | 2,19 | 2,17 | 2,16 |
| FA | 0,30 | 0,27 | 0,12 | 0,05 | 0,015 | n.m. |
| tar | 0,32 | 0,19 | 0,15 | 0,05 | 0,02 | 0,02 |

n.m.=not measurable

Example VII

Example I was repeated with butadiene as the additive. To this end two sample bottles were charged with aniline and hydrochloric acid, and the content of one of these bottles was then exposed to a stream of butadiene gas at atmospheric pressure during 15 minutes.

On closing the bottle and shaking its content a total of about 2 wt.% of butadiene appeared to have been absorbed.

Subsequently, after adding 1,0 ml of a 40% $NaNO_2$ solution in water the further procedure was carried out keeping the bottle tightly closed.

The analytical data obtained are summarized in Table F.

TABLE F

| | additive | |
|---|---|---|
| | none | butadiene |
| pAAB | 26,1 | 27,7 |
| oAAB | 2,02 | 1,97 |
| FA | 0,4 | 0,05 |
| tar | 0,24 | <0,03 |

**Claims**

1. A process for the preparation of p-aminoazobenzene which comprises the steps of reacting an excess of aniline with an alkali metal nitrite in a hydrochloric acid medium at a temperature below 100°C, isomerizing the resulting diazoamino compound in the same medium, neutralizing the reaction mixture and separating the resulting aqueous phase from the organic phase which contains the formed p-aminoazobenzene dissolved in aniline, characterized in causing the isomerization to proceed in the presence of a compound selected from the group consisting of acrylonitrile, methacrylonitrile, methacrylic acid, alkyl methacrylate with 1 to 4 carbon atoms in the alkyl radical, and butadiene in an amount of at least 0.1% by weight, based on the aniline.

2. A process according to claim 1, wherein the selected compound is present in an amount of 0,5 to 10% by weight, based on the aniline.

**Patentansprüche**

1. Verfahren zur Herstellung von p-Aminoazobenzol, umfassend die Stufen eines Umsetzens eines Überschusses an Anilin mit einem Alkalimetallnitrit in einem Chlorwasserstoffsäuremedium bei einer Temperatur unter 100°C, eines Isomerisierens der erhaltenen Diazoaminoverbindung in demselben Medium, eines Neutralisierens des Reaktionsgemisches und eines Abtrennens der erhaltenen wäßrigen Phase von der organischen Phase, die das gebildete, in Anilin gelöste p-Aminoazobenzol enthält, dadurch gekennzeichnet, daß man die Isomerisierung in Gegenwart einer Verbindung, die aus der Gruppe Acrylnitril, Methacrylnitril, Methacrylsäure, Methacrylsäurealkylester mit 1 bis 4 Kohlenstoffatomen im Alkylrest und Butadien ausgewählt ist, in einer Menge von mindestens 0,1 Gewichtsprozent, bezogen auf das Anilin, vorgehen läßt.

2. Verfahren nach Anspruch 1, wobei die ausgewählte Verbindung in einer Menge von 0,5 bis 10 Gewichtsprozent, bezogen auf das Anilin, vorliegt.

**Revendications**

1. Procédé de préparation de p-aminoazobenzène qui comprend les étapes suivantes: réaction d'un excès d'aniline avec un nitrite de métal alcalin en milieu acide chlorhydrique à une température inférieure à 100°C, isomérisation du composé diazoamino résultant dans le même milieu, neutralisation du mélange réactionnel et séparation de la phase aqueuse résultante de la phase organique qui contient le p-aminoazobenzène dissous dans l'aniline, caractérisé en ce que l'isomérisation est effectuée en présence d'au moins 0,1% en poids, par rapport à l'aniline, d'un composé choisi parmi l'acrylonitrile, le méthacrylonitrile, l'acide méthacrylique, les méthacrylates, d'alkyle, dont le radical alkyle contient entre 1 et 4 atomes de carbone, et le butadiène.

2. Procédé selon la revendication 1, caractérisé en ce que le composé choisi est présent en quantité de 0,5 à 10% en poids, par rapport à l'aniline.